Europäisches Patentamt

**(19)** European Patent Office

Office européen des brevets

**(11)** Publication number: **0 295 961**
**A2**

**(12)** # EUROPEAN PATENT APPLICATION

**(21)** Application number: 88305582.4

**(22)** Date of filing: 17.06.88

**(51)** Int. Cl.⁴: **A 61 K 35/66**
A 61 K 35/70, A 61 K 35/72,
A 61 K 35/74, A 61 K 35/84,
C 12 P 21/00

**(30)** Priority: 18.06.87 JP 152085/87
18.06.87 JP 152089/87
18.06.87 JP 152090/87
18.06.87 JP 152091/87

**(43)** Date of publication of application:
21.12.88 Bulletin 88/51

**(84)** Designated Contracting States: BE DE FR GB IT

**(71)** Applicant: **KUREHA KAGAKU KOGYO KABUSHIKI KAISHA**
**1-9-11, Nihonbashi, Horidome-cho**
**Chuo-ku Tokyo 103 (JP)**

**(72)** Inventor: Hirose, Kunitaka
3-26-1 Hyakunin-cho Shinjuku-ku
Tokyo (JP)

Furusho, Takao
1-6-13 Asahi-machi
Michida-shi Tokyo (JP)

Muto, Shigeaki
3-23-2, Higashi-Horikiri
Katsushika-ku Tokyo (JP)

Niimura, Koichi
6-104 Shin-Sayama-Haitsu 63 Aoyagi
Sayama-shi Saitama-ken (JP)

Oohara, Minoru
19-25 Fujima-cho Itabashiku
Tokyo (JP)

Oguchi, Yoshiharu
306 Koopo-Cherii 3-10-23 Kasuga-cho
Nerima-ku Tokyo (JP)

Matsunaga, Kenichi
989-17 Kami-Arai
Tokorozawa-shi Saitama-ken (JP)

Kakuchi, Junji
6-1-30-406 Minami-Karasuyama
Setagaya-ku Tokyo (JP)

Sugita, Norifumi
2-10-13-402 Kasuga Bunkyo-ku
Tokyo (JP)

Yoshikumi, Chikao
2-19-46 Higashi
Kunitachi-shi Tokyo (JP)

Takahashi, Masaaki
1-5-33-314 Takanawa
Minato-ku Tokyo (JP)

**(74)** Representative: **Woods, Geoffrey Corlett**
**J.A. KEMP & CO. 14 South Square Gray's Inn**
**London WC1R 5EU (GB)**

**(54)** Polysaccharides and antiviral drug containing polysaccharides as active ingredient.

**(57)** A polysaccharide produced from a procaryotic or eucaryotic organism and useful as an antiviral agent, especially an antiretroviral agenct, has the following properties:

(a) (i) positive results when subjected to the α-naphthol-sulfuric acid, indole-sulfuric acid, anthrone-sulfuric acid or phenol-sulfuric acid reaction, or
(ii) positive results when subjected to the α-naphthol-sulfuric acid, indole-sulfuric acid, anthrone-sulfuric acid or phenol-sulfuric acid reaction and, after hydrochloric acid hydrolysis, to the Lowry-Folin process or ninhydrin reaction;

(b) elementary analysis: 20-55% carbon, 3-9% hydrogen and less than 16% nitrogen;

(c) a pH of 6.0-7.5;

(d) an infrared absorption spectrum peak at
(i) 3600-3200 cm⁻¹ or
(ii) 3600-3200 cm⁻¹ and 1700-1600 cm⁻¹;

(e) a molecular weight of from $10^3$ to $3 \times 10^6$ as measured by gel filtration chromatography; and

(f) soluble in water and aqueous solvents containing water-soluble alcohols, acids or bases and insoluble in chloroform, benzene and ether.

EP 0 295 961 A2

## Description

## POLYSACCHARIDES AND ANTIVIRAL DRUG CONTAINING POLYSACCHARIDES AS ACTIVE INGREDIENT

The present invention relates to polysaccharides obtained from the fungi and an antiviral drug containing polysaccharides as active ingredient.

The term "polysaccharides" used as active ingredient of the antiviral drug according to the present invention means a polysaccharide and a protein-bound polysaccharide which are obtainable from the fungus species of Procaryomycetes, Eucaryomycetes, Ascomycetes and Basidiomycetes belonging to the families Ganodermeae, Tricholomateceae and Auriculariiaceae.

In recent years new types of viral diseases such as B type hepatitis, adult T cell leukemia and AIDS (acquired immunodeficiency syndrome) have been attracted. Human being has combatted the viral diseases by means of vaccination and succeeded in eradicating such diseases as smallpox, yellow fever and polio. However, vaccination alone is not enough to cope with the diseases involving the problem of persistent infection or cryptogenic infection such as AIDS, and thus the development of an antiviral drug which is safe and highly efficacious has been expected.

The present inventors had previously proposed the polysaccharides extracted from the fungi of Basidiomycetes and having an anticancer effect. For instance, in British Patent No. 1,331,513 is proposed polysaccharides obtained from a liquid extract of a mycelium of a fungus speoies of Basidiomycetes or from a cultured broth of the species, said polysaccharide being free or substantially free of impurities originally present in the liquid extract or in the cultured broth, and being characterized by being a water-soluble amorphous solid which is non-hydroscopic and non-dialyzable; gives a positive result when tested for the presence of glucose after hydrolysis with 1N sulphuric acid; gives negative results when subjected to the ferric chloride reaction for determining the presence of phenols and to the Fehling reaction for determining the presence of reducing sugars; gives positive results when subjected to the anisaldehyde-sulphuric acid reaction, the Molisch reaction with $\alpha$-naphthol, the anthrone-sulphuric acid reaction, the tryptophane-sulphuric acid reaction, chromotropic acid-sulphuric acid reaction, the aniline-hydrochloric acid reaction, the resorcinol-hydrochloric acid reaction, the carbazole-cysteine-sulphuric acid reaction, the Tollens reaction and the thioglycol-sulphuric acid reaction; shows only one spot at the anode side when subjected to electrophoresis in a 0.05 mol. sodium borate solution for 90 minutes using a cellulose acetate membrane at 20 - 25 V/cm; and shows no antimicrobial action to bacteria, fungi and yeasts such as Staphylococcus aureus, Escherichia coli, Bacillus subtilis, Aspergillus niger and Candida albicans.

As a result of further studies of the present inventors for providing a safe and efficacious antiviral drug, it has been found that the polysaccharides produced from the fungus species of Procaryomycetes, Eucaryomycetes, Ascomycetes and Basidiomycetes belonging to the families Ganodermeae, Tricholomateceae and Auriculariaceae have an inhibiting activity against adsorption of human immuno-deficiency virus (HIV) on cells as well as a function to inhibit an activity of the reverse transcriptase. The present invention has been attained on the basis of such finding.

In a first aspect of the present invention, there is provided polysaccharides produced from the fungus species of Procaryomycetes, Eucaryomycetes and Ascomycetes, having the following properties:

(a) (i) positive results when subjected to the $\alpha$-naphthol-sulfuric acid reaction, indole-sulfuric acid reaction, anthrone-sulfuric acid reaction or phenol-sulfuric acid reaction, or

(ii) positive results when subjected to the $\alpha$-naphthol-sulfuric acid reaction, indole-sulfuric acid reaction, anthrone-sulfuric acid reaction or phenol-sulfuric acid reaction and Lowry-Folin process or ninhydrin reaction after hydrochloric acid hydrolysis;

(b) an elementary analysis of 20 - 55% carbon, 3 - 9% hydrogen and less than 16% nitrogen;

(c) a pH of 6.0 - 7.5;

(d) (i) an absorption peak at 3600 - 3200 cm$^{-1}$ or

(ii) absorption peaks at 3600 - 3200 cm$^{-1}$ and 1700 - 1600 cm$^{-1}$ in the infrared absorption spectrum;

(e) a molecular weight of $10^3$ - $3 \times 10^6$ as measured by gel filtration chromatography; and

(f) soluble in water and aqueous solvents containing water-soluble alcohols, acids or bases and insoluble in organic solvents of chloroform, benzene and ether.

In a second aspect of the present invention, there is provided an antiviral drug containing as an active ingredient an effective amount of polysaccharides produced from the Procaryomycetes, Eucaryomycetes, Ascomycetes and Basidiomycetes of the families Tricholomateceae, Ganodermeae and Auriculariaceae.

The characteristic of the present invention lies in polysaccharides extracted from the fungus species of Procaryomycetes, Eucaryomycetes and Ascomycetes and having an inhibiting activity against adsorption of human immunodeficiency virus on human lymphocytes as well as a function for inhibiting an activity of the reverse transcriptase (said polysaccharides being hereinafter referred to as the present substance (A)), and an antiviral drug containing as an active ingredient polysaccharides extracted from the fungus species of Procaryomycetes, Eucaryomycetes, Ascomycetes and Basidiomycetes of the families Ganodermeae, Tricholomateceae and Auriculariaceae (these polysaccharides being hereinafter referred to as the present substance (B)).

The fungi used in the present invention are defined and identified in "IWANAMI ENCYCLOPEDIA BIOLOGICA", 3rd Ed. (published March 10, 1983, by Iwanami Shoten); Yoshimura: Colored Illustration of Japanese Lichen (published July 1, 1977, Hoikusha); R. Imazeki and T. Hongo: Colored Illustration of Fungi of Japan (Hoikusha); and S. Ito: Handbook of Japanese Fungi (Yokendo).

The Procaryomycetes usable in the present invention include bacteria, myxobacteria and actinomycetes. The Eucaryomycetes usable in the present invention include myxomycetes, acryasiomycetes, oomycetes, chytridiomycetes, zygomycetes and dueteromycetes. The Ascomycetes usable in the present invention include lichen of the order Lecanorales, Sphaeriales, Caliciales, Hysteriales, Pleosporales and Myriangiales of the Ascolichenes.

The Basidomycetes used in the present invention is fungi belonging to the families Ganodermeae, Tricholomateceae and Auriculariaceae.

Among these fungi, those belonging to the genera Enterococcus, Pseudomonas, Lactobacillus, Aspergillus, Saccharomyces, Mucor, Gyrophora, Lecanactis, Calicium, Ganoderma, Flammulina and Auricularia are preferred.

For obtaining an artificial culture of a fungus used in the present invention, the fungus is inoculated into a medium and cultured at a proper temperature. Usually a liquid medium is preferably used in view of easiness of treatment and high productivity.

A medium composition used for ordinary culture is sufficient for culture of the fungus used in the present invention, that is, it is enough if the medium contains the nutrients necessary for the growth of the fungus. Regarding the nutrients, as carbon source dextrose, maltose, lactose, sucrose, starch, blackstrap molasses and the like are usable, and as nitrogen source peptone, extract, yeast extract, yeast, corn steep liquor, ammonium salts, organic and inorganic nitrogen compounds such as urea and the like can be used. Inorganic salts such as phosphate, magnesium salt, iron salt, calcium salt, common salt, etc., may be added as mineral component. Other substances such as serum L-cystine hydrochloride, sodium thioglycolate and vitamins necessary for the growth of the fungus may be added.

Culture is usually carried out at a temperature of 20 - 35°C for 2 to 30 days with an initial pH of 2 to 7. A pH of 4 to 7 and a temperature of 25 to 30°C are preferred for the culture in the present invention. In the case of aerated spinner culture, it is preferably carried out at an aeration rate of 0.1 to 2.0 l/min and a stirring speed of 30 to 800 r.p.m.

The present substances (A) and (B) can be obtained (i) by extracting a fungus with an aqueous solvent and subjecting the extract to refining treatment, or (ii) by subjecting the supernatant of a cultured broth thereof to refining treatment.

The aqueous solvent used for the extraction of the fungus is selected from water and aqueous solutions containing a small amount, such as not more than 10%, of an organic solvent, acid, base or salt soluble in water. The extraction according to the present invention is carried out a method selected from the group consisting of the water extraction, the organic-solvent extraction, the acid-solution extraction, the base-solution extraction, the salt extraction and a combination thereof. The organic solvents usable in the present invention in clude methanol, ethanol, isopropyl alcohol and the like. Hydrochloric acid, sulfuric acid, acetic acid and the like can be used as acid contained in the aqueous solutions. The bases usable for the aqueous solutions include ammonia, sodium hydroxide, potassium hydroxide, sodium carbonate and the like. Sodium chloride, potassium chloride and the like can be used as salt. The extraction according to the present invention is usually carried out at a temperature of 4 to 120°C for a period of 20 minutes to 20 hours by using 5 to 200 times as much amount of extracting solution as the fungus (dry basis).

The refining treatment according to the present invention is intended to remove the low-molecular weight substances by applying such treatment as salting-out, dialysis, ultrafiltration, reverse osmosis, gel filtration, precipitation by use of an organic solvent, etc., which may be conducted either singly or in combination. Technologically, it is preferred to conduct either ultrafiltration, reverse osmosis or a combination thereof, which are the pressurized membrane separation methods. In some cases, such treatment(s) may be conducted after salting-out.

Ammonium sulfate, common salt (sodium chloride), potassium chloride, barium carbonate and the like can be used as salting-out agent, ammonium sulfate being preferred. The salting-out step needs to be followed by at least one treatment selected from dialysis, ultrafiltration, gel filtration and reverse osmosis.

Dialysis is usually conducted by using a semi-permeable membrane such as cellophane or collodion membrane.

Gel filtration is carried out by using a column packed with an adsorbent such as dextran, polyacrylamide gel or the like. The fillers commercially available under the trade names of Cephadex and Bio-Gel are usually used.

Ultrafiltration and reverse osmosis are both methods for fractionation using a membrane under pressure. The ultrafiltration is usually carried out under a pressure of 0.5 to 5 kg/cm$^2$ and the reverse osmosis is carried out under a pressure of 20 to 35 kg/cm$^2$.

Precipitation by use of an organic solvent is generally conducted by using such solvent as methoanol, ethanol, isopropanol, acetone or the like. If necessary, an ion exchange treatment may be applied in addition to said operations.

The refined product is dehydrated by spray-drying, freeze-drying or other means and worked into a final product.

In case of obtaining the present substances (A) and (B) from a cultured medium, the refining treatment alone may be used.

The present substances (A) and (B) obtained by extracting a specified fungus with water or an aqueous solution containing a small amount of an organic solvent, acid or salt and subjecting the extract to refining treatment are preferably further treated with an aqueous alkaline solution since the antiviral effect of the thus treated substances are surprisingly increased by this alkaline solution treatment.

Such aqueous alkaline solution treatment can be effected, for example, by treating the present substances (A) and (B) with 5 to 200 times as much amount of a 0.01 - 5N, preferably 0.1 - 2N aqueous alkaline solution at a temperature of 40 - 250°C, preferably 60 - 200°C, more preferably 100 - 150°C, for 5 minutes to 2 hours, preferably 10 minutes to one hour. Then the treated solution is neutralized and subjected to refining treatment selected from salting-out, dialysis, ultrafiltration, reverse osmosis, gel filtration, precipitation by use of an organic solvent and a combination thereof. The conditions for these refining treatment are the same as described above. After the refining treatment, the resulting product is dehydrated by spray drying, freeze drying or other means.

The present substances (A) and (B) obtained in the manner described above (i) give positive results when subjected to the $\alpha$-naphthol-sulfuric acid reaction, indole-sulfuric acid reaction, anthrone-sulfuric acid reaction or phenol-sulfuric acid reaction (polysaccharide) or (ii) give positive results or weakly positive results when subjected to the $\alpha$-naphthol-sulfuric acid reaction, indole-sulfuric acid reaction, anthrone-sulfuric acid reaction or phenol-sulfuric acid reaction and Lowry-Folin process or ninhydrin reaction after hydrochloric acid hydrolysis (protein-bound polysaccharide).

The elementary analysis of the present substances (A) and (B) shows 20 - 55% carbon, 3 - 9% hydrogen and less than 16.0% nitrogen, and a pH of 6.0 - 7.5.

The infrared spectrum of the present substances (A) and (B) shows (i) absorption of hydroxyl group at 3600 - 3200 cm$^{-1}$ (polysaccharide) and shows (ii) absorption of hydroxyl group at 3600 - 3200 cm$^{-1}$ and absorption attributable to amide group at 1700 - 1600 cm$^{-1}$ (protein-bound polysaccharide).

The present substances (A) and (B) are soluble in aqueous solvents and insoluble in organic solvents. The "aqueous solvents" referred to herein include water and aqueous solutions containing alcohols, acids, bases, etc., which are soluble in water. The "organic solvents" refer to such solvents as chloroform, benzene, ether and the like.

The present substances (A) and (B) are white or brown in color and have a molecular weight of $10^3 - 3 \times 10^6$ as measured by gel filtration chromatography.

In an acute toxicity test of the present substances (A) and (B) in which the present substances were orally administered to the rats (Donryu strain) 4 - 5 weeks old and having a body weight of 100 - 150 g at a dose of 1000 mg/kg and their conditions were observed for seven days, all of the tested rats were alive through the test period. The present substances (A) and (B) are a safe material which is very low in toxicity and causes almost no harmful side effect.

It is known that generally a virus is adsorbed on a target cell and nucleic acid of the virus is injected into the cell and further integrated into the genome of the cell, and through this process the virus is replicated. In the case of retrovirus, a process of transfering RNA, which is a virus-derived nucleic acid, into DNA by the action of reverse transcriptase is necessary before the nucleic acid is integrated into the genome of the cell.

The present substances (A) and (B) have a function to inhibit adsorption of HIV (human immunodeficiency virus) on human lymphocytes and succeeding infection thereof and a function to inhibit an activity of the reverse transcriptase. The actual effect of the present substances (A) and (B) was examined by a method in which HIV (human immunodeficiency virus) which had been treated with the 400 µg/ml conc. solutions of the present substances (A) and (B) (the compounds of Examples 1 - 20) at 0°C for 2 hours and then washed was applied to the MT-4 cells to infect them with the virus, and after 3-day culture, the number of the HIV antigen positive cells was counted. It was found that the HIV antigen positive cells have been substantially eliminated by the pretreatment with the present substances (A) and (B), indicating the strong inhibiting activity against adsorption of HIV on human lymphocytes. When the effect of the present substances (A) and (B) on the reverse transcriptase activity was examined by using whole messenger RNA from rat liver as template, a strong inhibition against reverse transcriptase activity was seen at a concentration of 500 µg/ml of the present substances (A) and (B).

These facts attest to the inhibiting function of the present substances (A) and (B) against viral infection, especially against the infection of retroviruses having reverse transcriptase, and particularly against AIDS caused by HIV infection.

In the case of azido-3'-dioxythymidine (AZT) which is already used as an antiviral drug, it produces a side effect of inhibiting segmentation of even normal cells, whereas the present substances (A) and (B) are a safe material which is extremely low in acute toxicity and useful as an antiviral drug since the present substances (A) and (B) show an inhibiting activity against virus infection especially retroviruses infection. Thus, the present substances are effective for the treatment of viral infectious diseases, especially retroviral infectious diseases such as AIDS.

In use of the present substances (A) and (B) as an antiviral drug, they can be worked into any desired form of preparation. Also, the drug can be administered in various ways. Further, the present substances (A) and (B) can be used in combination with other known antiviral drugs such as AZT mentioned above without lowering their efficacy. Such combined use with other drugs is indeed an effective means for the treatment of the diseases.

In the case of oral administration, the drug may take the form of tablet, granules, powder, capsule, etc., which may contain in their composition an adjuvant or adjuvants usually used for the pharmaceutical

4

preparations, such as binder, inclusion, excipient, lubricant, disintegrator, wetting agent, etc. In case the present substances are used as a liquid preparation for oral administration, the preparation may take the form of liquid medicine for internal use, shake mixture, suspension, emulsion, syrup, etc. It may take the form of a dry product which is dissolved in water when used. Also, such liquid preparations may contain the usually used adjuvant(s) and preservative(s). In the case of injection, the composition may contain such additives as stabilizer, buffer agent, preservative, isotonizing agent, etc., and is offered in the form of unit-dose ampule or in the multiple-dose containers. Also, the composition may take the form of aqueous solution, suspension, solution, emulsion in an oleaginous or aqueous vehicle, etc. The active ingredient (the present substances) may be provided as a powder which, when used, is dissolved in a suitable vehicle such as pyrogen-free sterilized water.

The antiviral drug according to the present invention is administered to man and animals either orally or parenterally. Oral administration includes sublingual administration, and parenteral administration includes injection such as subcutaneous, intramuscular and intravenous injection and instillation. The dosage of the antiviral drug according to the present invention is variable depending on whether the subject is man or animal and also according to the age, individual difference, condition of the disease and other factors, but usually in the case where the subject is man, the oral dose of the present substances (A) and (B) is 0.1 to 1,000 mg, preferably 1 to 100 mg, per kg of body weight and day, and this amount of the present substances is given in one to three portions.

The present substances (A) and (B) are a material having an inhibiting activity against HIV adsorption as well as a function to inhibit a reverse transcriptase activity, and showing extremely low in acute toxicity and high in safety. Further, the antiviral drug according to the present invention is effective for the treatment of viral infectious diseases, especially retroviral infectious diseases such as AIDS.

The present invention is explained in more detail in the following Examples; however, it should be recognized that the scope of the present invention is not restricted to these Examples.

Example 1

Enterococcus faecalis (IAM-1262) was cultured in 10 litres of a medium composed of 0.1% of meat extract, 0.5% of sodium chloride and 1% of peptone. The culture was centrifuged at 4,000 x g' for 30 minutes to separate the mycelia from the supernatant.

The supernatant was concentrated under reduced pressure, then dialyzed with a visking cellulose tube to remove the low-molecular substances and freeze-dried to obtain 30 g of a brown dry product.

Example 2

The mycelia obtained in Example 1 was suspended as uniformly as possible in 5 times as much volume of a physiological saline solution under stirring. This suspension was centrifuged at 4,000 x g, for 30 minutes. The supernatant was discarded. The precipitate was suspended in a physiological saline solution and the cells were broken by using a French press. After confirming that substantially all of the cells have become void, the suspension was centrifuged at 4,000 x g' for 30 minutes and the residue was again suspended in water. The above operations were repeated thrice.

After washing, the residue was put into ethanol and centrifuged. After replacement with 100% ethanol and acetone, the product was dried under reduced pressure to obtain 23 g of powder.

Example 3

10 g of the fungus extract obtained in Example 2 was added into 200 ml of a 1N sodium hydroxide solution and heat-treated in an autoclave at 120°C for 20 minutes. The mixture was adjusted to a pH of 7 and dialyzed with a visking cellulose tube to remove the low-molecular substances such as salt.

The remaining material was freeze-dried to obtain 8.4 g of a dry product.

Example 4

A mycelia of Pseudomonas aeruginosa (IAM-1514) obtained by 4,000 x g' and 30-minute centrifuging of a culture of said fungus was suspended as uniformly as possible in 5 times as much volume of a physiological saline solution under stirring. This suspension was centrifuged at 4,000 x g' for 30 minutes. The supernatant was discarded and the precipitate was suspended in a physiological saline solution, and then the cells were broken by using a French press. After confirming that substantially all of the cells have become void, the suspension was centrifuged at 4,000 x g' for 30 minutes and the residue was again suspended in water. The above operations were repeated thrice.

After washing, the residue was put into ethanol and centrifuged. After replacement with 100% ethanol and acetone, the product was dried under reduced pressure to obtain 19 g of powder.

The precipitate, after washed, was extracted with a 0.5N NaOH solution at 80°C for 2 hours. The extract solution was cooled to room temperature, adjusted to a pH of 7.0 with 2N HCl, desalted by ultrafiltration and then freeze-dried to obtain 15.3 g of a dry product.

The medium used for the culture was of the following composition:
Peptone    1%
Soybean peptone    0.3%
Preteose peptone    1%
Pulverized digestive serum    1.35%
Yeast extract    0.5%
Pulverized beef extract    0.22%
Pulverized liver extract    0.12%
Dextrose    0.3%
Potassium dihydrogenphosphate    0.25%
Sodium chloride    0.3%
Starch    0.5%
L-cystine hydrochloride    0.03%
Sodium thioglycolate    0.03%

Example 5

17 g of an extract (dry product) was obtained from Bifidobacterium infantis (ATCC 15697) by following the same procedure as Example 4.

Example 6

22 g of an extract (dry product) was obtained from Lactobacillus casei (IAM-1118) according to the same procedure as Example 4.

Example 7

Aspergillus giaucus (IAM 3005) (an aspergillus belonging to the order Moniliales) was cultured in 10 litres of a medium composed of 30% of glucose, 1.1% of corn steep liquor and 0.53% of urea. The culture solution was centrifuged at 4,000 x g' for 30 minutes to separate the mycelial cake from the supernatant.
The supernatant was concentrated under reduced pressure, dialyzed with a visking cellulose tube to remove the low-molecular substances and freeze-dried to obtain 18 g of a brown dry product.

Example 8

The mycelia separated in Example 7 was suspended as uniformly as possible in 5 times as much volume of a physiological saline solution under stirring. This suspension was centrifuged at 4,000 x g' for 30 minutes. The supernatant was discarded. The precipitate was suspended in a physiological saline solution and the cells were broken by using a French press. After confirming that substantially all of the cells have become void, the suspension was centrifuged at 4,000 x g' for 30 minutes and the residue was again suspended in water. The above operations were repeated thrice.
After washing, the residue was put into ethanol and centrifuged, and after replacement with 100% ethanol and acetone, the resultant product was dried under reduced pressure to obtain 18 g of powder.

Example 9

5 g of mycelia obtained in Example 8 was suspended in 100 ml of a 1N sodium hydroxide solution and heat-treated in an autoclave at 120°C for 20 minutes. The mixture was adjusted to a pH of 7 with a 2N hydrochloric acid solution and dialyzed with a visking cellulose tube to remove the low-molecular substances such as salt. The residual material in the tube was freeze-dried to obtain 2.9 g of a dry product.

Example 10

A mycelia obtained after 4,000 x g' and 30-minute centrifuging of a culture solution (10 litres of 2% sucrose PDA) of Saccharomyces cerevisiae (IAM-42077) (belonging to the order Saccharomycetales) was suspended as uniformly as possible in 5 times as much volume of a physiological saline solution under stirring. This suspension was centrifuged at 4,000 x g' for 30 minutes. The supernatant was discarded and the precipitate was suspended in a physiological saline solution, and then the cells were broken by using a French press. After confirming that substantially all of the cells have become void, the suspension was centrifuged at 4,000 x g' for 30 minutes and the residue was again suspended in water. The above operations were repeated thrice.
After washing, the residue was extracted with a 0.5N NaOH solution at 80°C for 2 hours. The extract solution was cooled to room temperature, adjusted to a pH of 7.0 with 2N HCl, desalted by ultrafiltration and then freeze-dried to obtain 9.2 g of a dry product.

## Example 11

11 g of an extract (dry product) was obtained from <u>Mucor apinescens</u> (IAM-6071) by following the same procedure as Example 10 (culture medium: 2% sucrose PDA).

## Example 12

100 g of <u>Gyrophora</u> belonging to the order Lecanorales was broken into fine chips, put into a 3-litre stainless steel tank, added with 2,000 ml of water and kept at 90 - 95°C under stirring. The mixture was subjected to extraction for about 3 hours and then cooled to room temperature.

The extract slurry was centrifuged to be separated into extract solution and residue. The residue was further extracted with 2,000 ml of 0.4N NaOH at 90 - 95°C for 3 hours, then cooled to room temperature, adjusted to a pH of 7.0 with 2N HCl and centrifuged into extract solution and residue.

The extract solutions were joined, concentrated to 400 ml by a vacuum concentrator, desalted by ultrafiltration and freeze-dried to obtain 26.0 g of a dry product.

## Example 13

100 g of chips of Collema complanatum belonging to the family Collemaceae of the order Lecanorales were treated according to the same procedure as Example 12 to obtain 11.2 g of a dry product.

## Example 14

100 g of Calicium japonicum belonging to the family Caliciaceae of the order Caliciales was broken into fine chips, put into a 3-litre stainless steel tank, added with 2,000 ml of water and stirred while maintaining the temperature at 90 - 95°C. The mixture was subjected to extraction for about 3 hours and then cooled to room temperature.

The extract slurry was centrifuged into extract solution and residue. The residue was further extracted with 2,000 ml of water at 90 - 95°C for 3 hours, cooled to room temperature and centrifuged into extract solution and residue.

The extract solutions were joined, concentrated to 400 ml by a vacuum concentrator and freeze-dried to obtain 14.3 g of a dry product.

## Example 15

2 g of the dry extract of Calicium japonicum obtained in Example 14 was dissolved in 1N NaOH and heat-treated at 120°C for 20 minutes. After the heat treatment, the solution was cooled to room temperature, adjusted to a pH of 7.0 with 6N HCl and then centrifuged to remove the insolubles. The supernatant was desalted by ultrafiltration and then freeze-dried to obtain 0.6 g of a dry product.

## Example 16

100 g of <u>Lecanactis</u> belonging to the family Lecanactis premmea of the order Hysteniales was broken into fine chips, put into a 3-litre stainless steel tank, added with 2,000 ml of water and stirred with the temperature kept at 90 - 95°C. The mixture was subjected to extraction for about 3 hours and then cooled to room temperature.

The extract slurry was centrifuged to be separated into extract solution and residue. The residue was further extracted with 2,000 ml of water at 90 - 95°C for 3 hours, then cooled to room temperature and centrifuged into extract solution and residue.

The extract solutions were joined, concentrated to 400 ml by a vacuum concentrator and freeze-dried to obtain 10.2 g of a dry product.

## Example 17

100 g of a dry mycelia of <u>Ganoderma</u> CM-359 (FERM-P-6060) was broken into fine chips, put into a 3-litre stainless steel tank, added with 2,000 ml of water and stirred while keeping the temperature at 90 - 95°C. The mixture was subjected to extraction for about 3 hours and then cooled to room temperature.

The extract slurry was centrifuged to be separated into extract solution and residue. The residue was further extracted with 2,000 ml of 0.4N NaOH at 90 - 95°C for 3 hours and the extract was cooled to room temperature, adjusted to a pH of 7.0 with 2N HCl and centrifuged to be separated into extract solution and residue.

The extract solutions were joined, concentrated to 400 ml by a vacuum concentrator, then ultrafiltered to remove the low-molecular substances and freeze-dried to obtain 12.5 g of a dry product.

## Example 18

100 g of fine chips of Auricularia CM-886 (FERM-P-1763) were treated according to the same procedure as Example 17 to obtain 15.1 g of a dry product.

## Example 19

100 g of a dry mycelia of Flammulina CM-601 (FERM-P-3045) was broken into fine chips, put into a 3-litre stainless steel tank, added with 2,000 ml of water and stirred while maintaining the temperature at 90 - 95°C. The mixture was subjected to extraction for about 3 hours and then cooled to room temperature.

The extract slurry was centrifuged to be separated into extract solution and residue. The residue was further extracted with 2,000 ml of water at 90 - 95°C for 3 hours, then cooled to room temperature and centrifuged to be separated into extract solution and residue.

The extract solutions were joined, concentrated to 400 ml by a vacuum concentrator and freeze-dried to obtain 28 g of a dry product.

## Example 20

5 g of the extract (dry product) of Flammulina obtained in Example 19 was dissolved in 200 ml of 1.0N NaOH and heat-treated at 120°C for 20 minutes. After the heat treatment, the solution was cooled to room temperature, adjusted to a pH of 7.0 with 6N HCl, dialyzed with a visking cellulose tube in flowing water for 2 days to remove the low-molecular substances and then freeze-dried to obtain 3.8 g of a dry product.

The physicochemical properties of the present substances obtained in Examples 1 - 20 are shown in Table 1. In the table, the phenol-sulfuric acid color reaction indicates the presence of sugars, and the Lowry-Folin reaction indicates the presence of peptide bond. As for the molecular weight, the molecular weight distribution of the fraction rich with the present substance as measured by the gel filtration method was shown.

## Example 21

For examining the inhibiting activity of the present substances obtained in Examples 1 - 20, the inhibition effect of reverse transcriptase specifically retained by the retroviruses was determined according to the following method.

10 mg of a freeze dried sample of each of the present substances were dissolved in 10 ml of sterilized distilled water (concentration: 1 mg/ml).

Separately, 1 $\mu$l of 20 mM D.T.T. (dithiothreitol, produced by Sigma Co., Ltd.), 5 $\mu$l of a 5-fold concentrated enzyme reaction solution (250 mM Tris-Hcl (pH 8.3), 250 mM KCl and 40 mM $MgCl_2$), 1 $\mu$l of 3d NTP solution (1 mM dATp, 1 mM GTP and 1 m dTTP, produced by Sigma Co., Ltd.), 2 $\mu$l of 100 $\mu$g/ml oligomer (dT)$_{12-18}$ (produced by PL-Biochemicals Co., Ltd.), 1 $\mu$l of messenger RNA (derived from normal rat liver, 1 $\mu$g/$\mu$l), 0.5 $\mu$l of RNase inhibitor (16 unit/$\mu$l, produced by Takara Shuzo KK) and 1 $\mu$l of [$\alpha$-$^{32}$P] dCTP (approx. 800 ci/mmol, 10 $\mu$ci/$\mu$l, produced by Amasham Japan Co., Ltd.) were added into a 1.5 ml Eppendolf tube and the tube was placed in a 37°C water bath.

5 minutes thereafter, 12.5 $\mu$l of the previously prepared aqueous solution of present substance (concentration: 1 mg/ml) was added into the reaction tube. Then 1 $\mu$l of reverse transcriptase (7 unit/$\mu$l, derived from Rous associated virus, produced by Takara Shuzo Kabushiki Kaisha) was added and the mixture was reacted at 37°C so that the final amount of the reaction solution would become 25 $\mu$l.

One hour thereafter, 5 $\mu$l of the reaction solution was infiltrated into the 2 cm x 2 cm sheets of DEAE filter paper (produced by Toyo Roshi Kabushiki Kaisha). After air drying, each sheet was dipped in 10 ml of a 0.5M $Na_2HPO_4$ solution and [$\alpha$-$^{32}$P] dCTP which has been not used for DNA synthesis and remained on the sheet was washed away under shaking. (This operation was conducted 5 times at a five minutes' interval.)

Thereafter, each of said DEAE paper sheets was placed in a glass vial containing 10 ml of a liquid scintillation cocktail (made by Amersham Japan Co., Ltd.) and the radioactivity of each sheet was counted for one minute (c.p.m.) by a scintillation counter (made by Aroka Co., Ltd.).

The inhibiting rate (%) of the reverse transcriptase activity was determined from the following formula:

$$\text{Inhibiting rage of reverse transcriptase activity (\%)} = \frac{Co - Cs}{Co} \times 100$$

Co: Radioactivity of the sheet when not added with the present substance
Cs: Radioactivity of the sheet when added with the present substance

The inhibiting rate of the reverse transcriptase (RTase) activity of the present substances are shown in Table 1.

### Example 22

The inhibiting activity of the present substances against adsorption of HIV (AIDS virus) on human lymphocytes was examined by the following method. (All the operations were conducted under an aseptic condition.)

1 ml of a suspension of HIV-1 (human immunodeficiency virus) and 1 ml of a solution of present substance (concentration: 800 µg/ml) were put into a test tube and the tube was placed still in ice. Two hours later, 1 ml of the virus suspension was taken out of the test tube and applied to the human lymphocyte-derived cell strain MT-4 (Jpn. J. Cancer Ros. (Gann), 28, 219-229 (1982)) so that the virus would be adsorbed on the cells at a multiplicity of infection (M.O.I.) ≒ 2. After centrifuging at 2,000 r.p.m. for 10 minutes, the supernatant was discarded and the deposited MT-4 cells were suspended in RPMI 1640 (Gibco Laboratories, NY) containing 20% of FCS so that the cell concentration would become $2 \times 10^5$ cell/ml.

The MT-4 cell suspension was pipetted in 100 µl portions into the 96-hole plates and cultured under the conditions of 5% $CO_2$ and 37°C. On the third day of culture, the HIV adsorbed cells and the non-adsorbed cells were calculated by the indirect fluorescent antibody technique.

The MT-4 cells were fixed by methanol treatment and reacted with the HIV-1 infected patient antiserum at 37°C. 30 minutes thereafter, the cells were washed with PBS and reacted with FITC-bonded rabbit antihuman IgG at 37°C.

500 MT-4 cells were observed under a fluorescence microscope and the fluorescent positive cells were counted as HIV-adsorbed cells and the fluorescent negative cells as non-HIV-adsorbed cells.

The HIV-1 (HTLV-III) adsorption inhibition rate (%) was determined from the following formula:

$$\text{HIV-1 adsorption inhibition rate (\%)} = \frac{\text{HIV-1-adsorbed cells}}{\text{HIV-1 adsorbed cells} + \text{non-HIV-1-adsorbed cells}} \times 100$$

The results are shown in Table 1.

### Preparation Example

300 mg of the present substance (Example 1) was filled in each of the #0 hard capsules by using an pressure type automatic filler to prepare the medicinal capsules.

Table 1

| Physico-chemical properties of extract | Present Substance | | | | | | | | |
|---|---|---|---|---|---|---|---|---|---|
| | Compound of Example 1 | Compound of Example 2 | Compound of Example 3 | Compound of Example 4 | Compound of Example 5 | Compound of Example 6 | Compound of Example 7 | Compound of Example 8 | Compound of Example 9 |
| Name of bacterium or fungus | Enterococcus | Enterococcus | Enterococcus | Pseudomonas | Bifidobacterium | Lactobacillus | Aspergillus | Aspergillus | Aspergillus |
| Deposit No. | IAM-1262 | IAM-1262 | IAM-1262 | IAM-1514 | ATCC 15697 | IAM-1118 | IAM-3005 | IAM-3005 | IAM-3005 |
| Material | Supernatant | Mycelial cake | Mycelial cake | Mycelial cake | Mycelial cake | Mycelial cake | Supernatant | Mycelial cake | Mycelial cake |
| Color | Light yellow | Light brown | Light brown | Light brown | Light brown | Light brown | Light yellow | Grayish white | Light brown |
| pH | 6.7 | 7.1 | 6.8 | 6.5 | 7.0 | 6.6 | 6.2 | – | 6.8 |
| Absorption in infrared region (cm$^{-1}$) | 3600–3200 1700–1600 | 3600–3200 1700–1600 | 3600–3200 1700–1600 | 3600–3200 1700–1600 | 3600–3200 1700–1600 | 3600–3200 1700–1600 | 3600–3200 1700–1600 | 3600–3200 1700–1600 | 3600–3200 1700–1600 |
| Phenol-sulfuric acid color reaction | + | + | + | + | + | + | + | + | + |
| Lowry-Folin color reaction | + | + | + | + | + | + | + | + | + |
| Molecular weight distribution | $5\times10^3$–$5\times10^4$ | $10^4$–$2\times10^5$ | $10^4$–$10^5$ | $5\times10^3$–$10^5$ | $5\times10^3$–$10^5$ | $5\times10^4$–$10^5$ | $10^4$–$10^6$ | $10^4$–$10^6$ | $10^4$–$10^6$ |
| Elemental analysis | C: 41.9 H: 5.8 N: 6.2 | C: 40.7 H: 5.5 N: 10.1 | C: 43.1 H: 4.9 N: 13.8 | C: 42.0 H: 4.9 N: 10.8 | C: 40.2 H: 5.8 N: 11.3 | C: 42.0 H: 4.9 N: 13.0 | C: 42.0 H: 6.4 N: 3.8 | C: 44.2 H: 6.0 N: 4.3 | C: 40.2 H: 6.1 N: 5.5 |
| Solubility in chloroform (25°C) | Insoluble | Insoluble | Insoluble | Insoluble | Insoluble | Insoluble | Insoluble | Insoluble | Insoluble |
| Solubility in water (at 25°C) | Soluble | Sparingly soluble | Sparingly soluble | Sparingly soluble | Sparingly soluble | Sparingly soluble | Sparingly soluble | Hardly soluble | Easily soluble |
| RTase activity inhibition rate (%) (*) | ++ | ++ | +++ | ++ | + | ++ | ++ | + | +++ |
| HIV adsorption inhibition rate (%) (*) | +++ | +++ | +++ | +++ | +++ | +++ | + | ++ | +++ |
| Protein Content (%) | 38.8 | 63.1 | 86.3 | 67.5 | 70.6 | 81.3 | 23.5 | 26.9 | 34.4 |
| Sugar Content (%) | 41.4 | 26.4 | 6.1 | 23.8 | 14.4 | 13.6 | 51.5 | 54.4 | 51.1 |

Table 1 (continued)

| Physico-chemical properties of extract | Present Substance | | | | | | | | |
|---|---|---|---|---|---|---|---|---|---|
| | Compound of Example 10 | Compound of Example 11 | Compound of Example 12 | Compound of Example 13 | Compound of Example 14 | Compound of Example 15 | Compound of Example 16 | Compound of Example 17 | Compound of Example 18 |
| Name of bacterium or fungus | Saccharomyces | Mucor | Gyrophora | Collema | Calicium | Calicium | Lecanactis | Ganoderma | Auricularia |
| Deposit No. | IAM-42077 | IAM-6071 | | Collema complanatum | Calicium japonicum | Calicium japonicum | | FERM P-6060 | FERM P-1763 |
| Material | Mycelial cake | Mycelial cake | Mycelial cake | Mycelial cake | Mycelial cake | Mycelial cake | Mycelial cake | Mycelial cake | Mycelial cake |
| Color | Light brown | Light brown | Light brown | Brown | Light brown | Brown | Light brown | Brown | Brown |
| pH | 7.0 | 6.6 | 7.0 | 7.0 | 6.2 | 7.1 | 6.3 | 7.1 | 6.8 |
| Absorption in infrared region (cm$^{-1}$) | 3600-3200 1700-1600 | 3600-3200 1700-1600 | 3600-3200 1700-1600 | 3600-3200 1700-1600 | 3600-3200 1700-1600 | 3600-3200 1700-1600 | 3600-3200 1700-1600 | 3600-3200 1700-1600 | 3600-3200 1700-1600 |
| Phenol-sulfuric acid color reaction | + | + | + | + | + | + | + | + | + |
| Lowry-Folin color reaction | + | + | + | + | + | + | + | + | + |
| Molecular weight distribution | $10^4 - 10^6$ | $10^4 - 10^6$ | $5 \times 10^3 - 1 \times 10^6$ | $5 \times 10^3 - 1 \times 10^6$ | $5 \times 10^3 - 1 \times 10^6$ | $5 \times 10^3 - 1 \times 10^6$ | $5 \times 10^3 - 1 \times 10^6$ | $10^3 - 10^5$ | $10^3 - 10^5$ |
| Elemental analysis | C: 45.6 H: 6.2 N: 8.9 | C: 40.5 H: 6.6 N: 8.2 | C: 42.5 H: 5.8 N: 4.2 | C: 44.8 H: 6.2 N: 3.8 | C: 48.0 H: 4.9 N: 3.2 | C: 40.9 H: 5.7 N: 6.0 | C: 46.2 H: 5.4 N: 2.6 | C: 48.2 H: 5.9 N: 3.8 | C: 40.2 H: 6.4 N: 4.2 |
| Solubility in chloroform (25°C) | Insoluble | Insoluble | Insoluble | Insoluble | Insoluble | Insoluble | Insoluble | Insoluble | Insoluble |
| Solubility in water (at 25°C) | Easily soluble | Easily soluble | Easily soluble | Easily soluble | Easily soluble | Easily soluble | Easily soluble | Easily soluble | Easily soluble |
| RTase activity inhibition rate (%) | ++ | ++ | ++ | ++ | + | +++ | + | ++ | ++ |
| HIV adsorption inhibition rate (%) | +++ | + | +++ | +++ | +++ | +++ | +++ | +++ | +++ |
| Protein Content (%) | 54.6 | 53.8 | 28.3 | 20.5 | 18.5 | 33.5 | 15.3 | 28.0 | 25.6 |
| Sugar Content (%) | 27.1 | 26.6 | 54.2 | 64.2 | 71.0 | 48.3 | 64.4 | 60.6 | 62.1 |

0 295 961

Table 1
(continued)

| Physico-chemical properties of extract | Present Substance | |
|---|---|---|
| | Compound of Example 19 | Compound of Example 20 |
| Name of bacterium or fungus | Flamm-ulina | Flamm-ulina |
| Deposit No. | FERM P-3045 | FERM P-3045 |
| Material | Mycelial cake | Mycelial cake |
| Color | Light brown | Light brown |
| pH | 6.0 | 7.1 |
| Absorption in infrared region (cm$^{-1}$) | 3600-3200 1700-1600 | 3600-3200 1700-1600 |
| Phenol-sulfuric acid color reaction | + | + |
| Lowry-Folin color reaction | + | + |
| Molecular weight distribution | $10^3$-$10^5$ | $10^3$-$10^5$ |
| Elemental analysis | C: 46.5 H: 6.1 N: 2.5 | C: 45.2 H: 5.8 N: 4.9 |
| Solubility in chloroform (25°C) | Insoluble | Insoluble |
| Solubility in water (at 25°C) | Easily soluble | Easily soluble |
| RTase activity inhibition rate (%) | ++ | +++ |
| HIV adsorption inhibition rate (%) | +++ | +++ |
| Protein Content (%) | 15.6 | 30.6 |
| Sugar Content (%) | 72.1 | 48.2 |

Note (*)  +++ : 61 - 100%
          ++ : 31 - 60%
          + : 10 - 30%

## Claims

1. A polysaccharide obtainable from a procaryotic or eucaryotic organism and having the following properties:

(a) (i) positive results when subjected to the α-naphthol-sulfuric acid, indole-sulfuric acid, anthrone-sulfuric acid or phenol-sulfuric acid reaction, or

(ii) positive results when subjected to the α-naphthol-sulfuric acid, indole-sulfuric acid, anthrone-sulfuric acid or phenol-sulfuric acid reaction and, after hydrochloric acid hydrolysis, to the Lowry-Folin process or ninhydrin reaction;

(b) elementary analysis: 20-55% carbon, 3-9% hydrogen and less than 16% nitrogen;

(c) a pH of 6.0-7.5;

(d) an infrared absorption spectrum peak at

(i) 3600-3200 cm$^{-1}$ or

12

(ii) 3600-3200 cm$^{-1}$ and 1700-1600 cm$^{-1}$;

(e) a molecular weight of from $10^3$ to $3 \times 10^6$ as measured by gel filtration chromatography; and

(f) soluble in water and aqueous solvents containing water-soluble alcohols, acids or bases and insoluble in chloroform, benzene and ether.

2. A process for the preparation of a polysaccharide as defined in claim 1, which process comprises (i) extracting a procaryotic or eucaryotic organism with an aqueous solvent and subjecting the extract to refining treatment or (ii) subjecting the supernatant of a broth in which a said organism has been cultured to refining treatment.

3. A process according to claim 2, wherein the said organism is a microorganism.

4. A process according to claim 3, wherein the procaryotic microorganism is a bacterium.

5. A process according to claim 4, wherein the bacterium is a myxobacterium or actinomycete.

6. A process according to claim 3, wherein the eucaryotic microorganism is a myxomycete, acrasiomycete, oomycete, chytridiomycete, zygomycete or deuteromycete.

7. A process according to claim 3, wherein the eucaryotic microorganism is a fungus.

8. A process according to claim 7, wherein the fungus is a species of Ascomycetes.

9. A process according to claim 8, wherein the said species of Ascomycetes is an Ascolichenes fungus.

10. A process according to claim 3, wherein the said microorganism is one belonging to the genera Enterococcus, Pseudomonas, Bifidobacterium, Lactobacillus, Aspergillus, Saccharomyces, Mucor, Gyrophora, Collema, Lecanactis or Calicium.

11. A process according to claim 3, wherein the said microorganism is Enterococcus faecalis IAM-1262, Pseudomonas aeruginosa IAM-1514, Bifidobacterium infantis ATCC 15697, Lactobacillus casei IAM-1118, Aspergillus giaucus IAM 3005, Saccharomyces cerevisiae IAM-42077, Muco apinescens IAM-6071, Gyrophora, Collema complanatum, Calcicium japonicum or Lecanactis premmea.

12. A process according to any one of claims 2 to 11, wherein the aqueous solvent is water or an aqueous solution containing a water-soluble organic solvent, acid, base or salt.

13. A process according to claim 12, wherein the aqueous solvent is aqueous ammonia or a sodium hydroxide, potassium hydroxide or sodium carbonate solution.

14. A process according to any one of claims 2 to 13, wherein the extraction is carried out at from 4 to 250°C for from 20 minutes to 20 hours by using 5 to 200 times as much of extracting solution as the said organism on a dry basis.

15. A process according to any one of claims 2 to 14, wherein a culture mycelium is extracted.

16. A process according to any one of claims 2 to 15, wherein the refining treatment is at least one treatment selected from salting-out, dialysis, ultrafiltration, reverse osmosis, gel filtration and precipitation by use of an organic solvent.

17. A pharmaceutical composition comprising a pharmaceutically acceptable carrier or diluent and, as active ingredient, a polysaccharide as defined in claim 1 or which has been produced by a process as claimed in any one of claims 2 to 16.

18. A polysaccharide as defined in claim 1 or which has been produced by a process as claimed in any one of claims 2 to 15 for use as an antiviral agent.

19. A polysaccharide according to claim 18 for use as an antiretroviral agent.

20. A polysaccharide according to claim 19 for use in the treatment of AIDS.

21. Use of a polysaccharide as defined in claim 1 in the preparation of a medicament for use as an antiviral agent.

22. The use according to claim 21, wherein the medicament is for use as an antiretroviral agent.

23. The use according to claim 22, wherein the medicament is for use in the treatment of AIDS.

24. The use according to any one of claims 21 to 23, wherein the polysaccharide has been produced by a process as claimed in any one of claims 2 to 16.

25. The use according to any one of claims 21 to 23, wherein the polysaccharide has been produced from a basidiomycete of the family Tricholomateceae, Ganodermeae or Auriculariaceae.

26. The use according to claim 25, wheein the basidiomycete belongs to the genera Ganoderma, Flammulina or Auricularia.

27. The use according to claim 26, wherein the basidiomycete is Ganoderma CM-359 (FERM-P-6060), Auricularia CM-886 (FERM-P-1763) or Flammulina CM-601 (FERM-P-3045).

28. The use according to any one of claims 25 to 27, wherein the polysaccharide has been obtained by (i) extracting a said basidiomycete with an aqueous solvent and subjecting the extract to refining treatment or (ii) subjecting the supernatant of a broth in which a said basidomycete has been cultured to refining treatment.

29. The use according to claim 28, wherein the aqueous solvent is water or an aqueous solution containing a water-soluble organic solvent, acid, base or salt.

30. The use according to claim 29, wherein the aqueous solvent is aqueous ammonia or a sodium hydroxide, potassium hydroxide or sodium carbonate solution.

31. The use according to any one of claims 28 to 30, wherein extraction is conducted at from 4 to 250°C for from 20 minutes to 20 hours by using 5 to 200 times as much of extracting solution as the said basidiomycete on a dry basis.

32. The use according to any one of claims 28 to 31, wherein a culture mycelium is extracted.

**0 295 961**

33. The use according to any one of claims 28 to 32, wherein the refining treatment is at least one treatment selected from salting-out, dialysis, ultrafiltration, reverse osmosis, gel filtration and precipitation by use of an organic solvent.

34. An antiviral drug containing as an active ingredient an effective amount of polysaccharides produced from the Procaryomycetes, Eucaryomycetes, Ascomycetes and Basidiomycetes of the families Tricholomateceae, Ganodermeae and Auriculariaceae.

35. An antiviral drug according to claim 34, wherein the polysaccharides are obtained by (1-1) extracting a fungus or bacterium with water or an aqueous solution containing a water-soluble organic solvent, acid or salt, and subjecting the extract, to refining treatment, or (1-2) subjecting a supernatant of the cultured broth thereof, (2) further treating the refined material with 5 to 200 times as much amount of a 0.01 - 5N aqueous alkaline solution at 40-250° C for 5 minutes to 20 hours, and (3) subjecting the treated material to refining treatment.

14